# EUROPEAN PATENT APPLICATION

(11) **EP 4 197 427 A1**
(43) Date of publication of application: **21.06.2023**
(21) Application number: 21306814.1
(22) Date of filing: 16.12.2021
(51) Int. Cl.: A61B 3/103, A61B 3/00

(54) **METHOD AND DEVICE FOR EVALUATING REFRACTION OF AN EYE OF AN INDIVIDUAL USING MACHINE LEARNING**

(71) Applicant: Essilor International, 94220 Charenton-Le-Pont (FR)
(72) Inventor: BOUTINON, Stéphane, 75013 PARIS (FR); PELOUX, Marius, 91120 PALAISEAU (FR); PINAULT, Philippe, 94130 NOGENT SUR MARNE (FR); DE ROSSI, Hélène, 94100 SAINT MAUR DES FOSSES (FR)
(74) Representative: Jacobacci Coralis Harle

(57) **Abstract**

The invention concerns a method (100) for estimating refraction of an eye of an individual using an image capturing device, the method comprising the following steps:
- acquiring (102) eccentric photorefraction images of the eye using the image capturing device when the eye is successively illuminated by a plurality of light sources;
- analyzing (104) the eccentric photorefraction images by a calculation module in order to determine at least one refraction parameter.

According to the invention, the step of analyzing (104) is carried out by machine learning using at least one neural network configured to determine the at least one refraction parameter from the eccentric photorefraction images, the plurality of light sources in the step of acquiring (102) being positioned at at least two different eccentric distances from the optical axis of the image capturing device and/or arranged along at least two different directions transverse to the optical axis of the image capturing device.

## Description

### TECHNICAL FIELD OF THE INVENTION

The invention relates to a method and a system for evaluating refraction of an eye of an individual.

More precisely the invention relates to a method and a device for estimating refraction of an eye of an individual. The invention also relates to a computer-program for estimating refraction of an eye of an individual. The method, device and/or and computer-program may be used for determining a prescription for ophthalmic lenses adapted for the individual or for manufacturing an ophthalmic lens according to the measured refraction. The invention also provides refraction evaluations that may be used as a starting point for further subjective refraction performed with another device.

### BACKGROUND INFORMATION AND PRIOR ART

Numerous documents describe devices and methods for measuring refraction of the eye of an individual. Subjective refraction methods are based on interactions with the individual viewing different optotypes and using a set of lenses with various refraction corrections. Objective refraction methods are based on measurements of the optical properties of the eye considered. In particular, some methods and devices for measuring objective refraction are based on eccentric photorefraction or photoretinoscopy.

Eccentric photorefraction is used to perform objective refraction by illuminating the user's eye using an eccentric light source and observing the image of the pupil with a camera. In most cases, reflected light forms on the pupil in the detected image, a light shape with a complementary, non luminous shape, called dark crescent, lunar or lunar lunch. The analysis of the size, shape and orientation of the bright or dark crescent enables to estimate refraction of the eye depending on the position of the eccentric light source. For example, the publications W. Wesemann, A.M. Norcia, D. Allen "Theory of eccentric photo refraction (photoretinoscopy): astigmatic eyes", J. Opt. Soc. Am. A, Vol. 8, No. 12, 1991, pages 2038-2047 or R. Kusel, U. Oechsner, W. Wesemann, S. Russlies, E. M. Irmer, and B. Rassow, "Light-intensity distribution in eccentric photorefraction crescents," J. Opt. Soc. Am. A 15, 1500-1511 (1998) disclose analytic expressions for the bright part. A rather simple method to deduce sphere, cylinder and axis values from measurements of a light gradient along three meridians is described in Gekeler F, Schaeffel F, Howland HC, Wattam-Bell J, "Measurement of astigmatism by automated infrared photoretinoscopy", Optometry and Vision Science : Official Publication of the American Academy of Optometry, 1997, Jul;74(7):472-482. DOI: 10.1097/00006324-199707000-00013.

However, these methods do not take into account higher order aberrations of the eye. Moreover, these publications are mostly theoretical, but do not disclose methods and systems enabling to quickly obtain refraction measurements. Also, depending on the user ametropia, it may be difficult to detect the crescent.

In addition, it is also known other methods to measure the refraction of the eye of an individual. These methods are functional but raise different problems, especially because they are time consuming and the result of the measurement lacks accuracy. For example, it is difficult with these methods to measure low levels of refractive correction.

There is a need for a system and method providing a measurement of photorefraction that is quick and very accurate, over a broad range of refraction values.

### SUMMARY OF THE INVENTION

One object of the invention is to provide a method for estimating refraction of an eye of an individual using an image capturing device, said image capturing device having an optical axis and being placed at a distance d from the eye of the individual, the method comprising the following steps:
- acquiring eccentric photorefraction images of the eye of the individual using the image capturing device when the eye is successively illuminated by a plurality of light sources, each eccentric photorefraction image corresponding to an image acquisition using at least one light source of the plurality of light sources;
- analyzing the eccentric photorefraction images by a calculation module in order to determine at least one refraction parameter comprising a sphere value.

According to the method of the invention, the step of analyzing is carried out by machine learning using at least one neural network configured to determine the at least one refraction parameter from the eccentric photorefraction images provided to the calculation module, the plurality of light sources in the step of acquiring being positioned at at least two different eccentric distances from the optical axis of the image capturing device and/or arranged along at least two different directions transverse to the optical axis of the image capturing device.

According to a particular and advantageous aspect of this method, the neural network is configured to determine the at least one refraction parameter based on the acquired eccentric photorefraction images and based on a set of inputs representing at least the distance d, and a position of each light source of the plurality of light sources relatively to the image capturing device.

By position, it is meant an eccentricity distance or distance and meridian angle.

Thus, the method according to the invention uses light sources arranged on different meridians allowing to perform, in a same time, a multi evaluations of the refraction parameters (or at least one refraction parameter) of the eye of the individual. The image capturing device does not need to be rotated, what improves the accuracy the evaluation. The method is further easy to implement because only one adjustment (for example the distance d of the acquiring, and the multievaluations) is needed in the method according to the invention. Then, having a neural network applied to multiple images corresponding to at least two different eccentric distances and/or two different known directions provides better results because the eye optics, such as retina, eye lens, cornea have orientations that depend on characteristics of the eye and because the eye has not a perfect revolution symmetric.

Consequently, the method according to the invention provides a method of the estimation of photorefraction that is quick, accurate and easy to implement.

According to another particular aspect, the sphere value comprises the equivalent sphere parameter, denoted M.

According to an embodiment, the method comprises a training step before the step of analyzing in order to train the at least one neural network based on a set of training eccentric photorefraction images stored in a database and an evaluation step to test the at least one neural network using a set of test eccentric photorefraction images stored in the same or another database, wherein each training eccentric photorefraction image of the set of training eccentric photorefraction images, and respectively, each test eccentric photorefraction image of the set of test eccentric photorefraction images, is associated with a value for each of the at least one refraction parameter.

According to a particular and advantageous aspect of this embodiment, the set of training eccentric photorefraction images and the set of test eccentric photorefraction images comprise images acquired using the image capturing device and/or simulated images obtained using a simulation model.

According to another embodiment, the method comprises a preprocessing step comprising an image recognition step configured to detect or discriminate elements from the eccentric photorefraction images, said elements being relative to areas of the eccentric photorefraction images.

According to a particular and advantageous aspect of this embodiment, the method further comprises a cropping step configured to select at least one of elements detected or discriminated in the image recognition step.

Advantageously, the at least one refraction parameter further comprises at least one other parameter of the eye among: astigmatism features, higher order aberrations, and/or wherein said step of analyzing further determining at least one individual parameter among: pupil size and shape of the eye of the individual, pupil diameter of the eye of the individual, half interpupillary distance, direction of gaze, amount of red reflex and Stiles-Crawford parameter.

According to a particular aspect, the at least one neural network used in the step of analyzing comprises a different neural network for each refraction parameter or a single neural network for all the refraction parameters of the at least one refraction parameter.

According to another particular aspect, the at least one neural network comprises a convolutional neural network comprising at least three convolutional layers and at least two output layers.

A further object of the invention is to provide a device for estimating refraction of an eye of an individual, said device for estimating refraction comprising:
- a plurality of light sources arranged to successively illuminate the eye of the individual;
- an image capturing device having an optical axis, the image capturing device being placed at a distance d from the eye of the individual, the image capturing device being arranged for capturing eccentric photorefraction images of the eye of said individual, each eccentric photorefraction image being associated to at least one light source of the plurality of light sources;
- a calculation module arranged to analyze the eccentric photorefraction images in order to determine at least one refraction parameter comprising a sphere value.

According to the device for estimating refraction of the invention, the calculation module is arranged to analyze the eccentric photorefraction images by machine learning using at least one neural network configured to determine the at least one photorefraction parameter, said plurality of light sources being positioned at at least two different eccentric distances from the optical axis of the image capturing device and/or arranged along at least two different directions transverse to the optical axis of the image capturing device.

The device according to the invention provides the same advantages of the method of the invention because the device according to the invention is easy to use and it provides quick and accurate results.

According to an embodiment, at least one light source of the plurality of light sources is at a distance comprised between 0,3 millimeter and 20 millimeters from an edge of the image capturing device, preferably from an edge of an aperture of the imaging capturing device.

According to another embodiment, each light source is placed at a distance from the other light sources comprised between 1 millimeter and 30 or 50 millimeters or spaced from the other light sources of an angle comprised between 3 degrees and 180 degrees, preferably between 3 degrees and 120 degrees, said angle being defined according to two different directions of two light sources of the plurality of light sources with respect to the optical axis of the image capturing device.

According to a particular aspect, the plurality of light sources is arranged to emit at a wavelength in the near infrared or infrared range.

Advantageously, the wavelength's peak emission is between 850 to 860 nanometers. In a particular aspect, each light source is adapted and configured to illuminate the eye with a pulse.

According to another particular aspect, the image capturing device is arranged to provide high-resolution eccentric photorefraction images.

According to an embodiment, the calculation module is embedded into a mobile device attached to the image capturing device or stored in a remote server.

A further object of the invention is to provide a computer-program product comprising one or more stored sequences of instructions that are accessible to a processor, and which, when executed by the processor, causes the processor to carry out the method according to the invention.

A further object of the invention is to provide a computer readable medium carrying one or more sequences of instructions of the computer program product of the invention.

### DETAILED DESCRIPTION OF EXAMPLE(S)

The following description with reference to the accompanying drawings will make it clear what the invention consists of and how it can be achieved. The invention is not limited to the embodiment/s illustrated in the drawings. Accordingly, it should be understood that where features mentioned in the claims are followed by reference signs, such signs are included solely for the purpose of enhancing the intelligibility of the claims and are in no way limiting on the scope of the claims.

In the accompanying drawings:
- Figure 1 shows a first embodiment of a method according to the invention;
- Figure 2 shows a second embodiment of a method according to the invention;
- Figure 3 shows a schematic representation of a pre-processing step of the method according to the invention;
- Figure 4 shows a first schematic representation of a neural network used in a method according to the invention;
- Figure 5 shows a second schematic representation of a neural network used in a method according to the invention;
- Figure 6 shows a third example of representation of a neural network used in a method according to the invention;
- Figure 7 shows a schematic representation of a device according to the invention.
- Figure 8A, respectively 8B, shows a perspective view of a device for estimating refraction of an eye of an individual according to a first, respectively second, embodiment of the invention;
- Figure 9 is an example of a front view of a photorefraction module of a device according to the invention comprising an image capturing device and a plurality of light sources for estimating refraction of an eye of an individual according to an embodiment of the invention;
- Figure 10 shows an exemplary configuration to carry out a method according to the invention.

In the description which follows the drawing figures are not necessarily to scale and certain features may be shown in generalized or schematic form in the interest of clarity and conciseness or for informational purposes. In addition, although making and using various embodiments are discussed in detail below, it should be appreciated that as described herein are provided many inventive concepts that may be embodied in a wide variety of contexts. Embodiments discussed herein are merely representative and do not limit the scope of the invention. It will also be obvious to one skilled in the art that all the technical features that are defined relative to a process can be transposed, individually or in combination, to a device and conversely, all the technical features relative to a device can be transposed, individually or in combination, to a process.

### Method

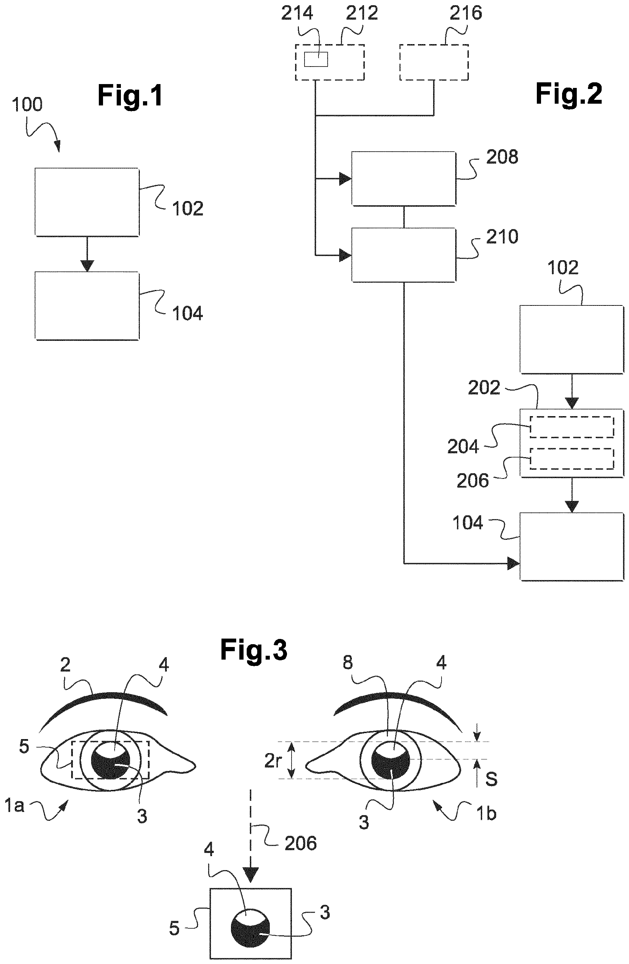

Figure 1 shows a first example of a method 100 according to the invention.

The method 100 is a method 100 for estimating refraction of an eye of an individual using an image capturing device. The image capturing device used in the method 100 has an optical axis and is placed at a distance d from the eye of the individual.

The method 100 comprises a step of acquiring 102 eccentric photorefraction images of the eye of the individual using the image capturing device. For each acquired eccentric photorefraction image, the eye of the individual is successively illuminated by one light source of a plurality of light sources. In the method 100, each eccentric photorefraction image corresponds to an image acquisition using one of the plurality of light sources. Thus each eccentric photorefraction image is associated to a light source belonging to the plurality of light sources.

The method 100 comprises a step of analyzing 104 the eccentric photorefraction images acquired in the step of acquiring 102 by a calculation module in order to determine at least one refraction parameter. The at least one refraction parameter comprises a sphere value corresponding to the spherical refraction parameter of the eye of the individual. In another embodiment, other refraction parameters are determined by the step of analyzing 104. For example, astigmatism parameters, such as the cylinder and the axis are determined.

In the method 100, the step of analyzing 104 is carried out by machine learning using at least one neural network configured to determine the at least one refraction parameter or all the refraction parameters (sphere value and astigmatism parameters) from the eccentric photorefraction images provided to the calculation module. In the method 100, the plurality of light sources used in the step of acquiring 102 are positioned at at least two different eccentric distances from the optical axis of the image capturing device and/or arranged along at least two different directions transverse to the optical axis of the image capturing device.

In this example, the neural network is already trained and it can be directly used in the step of analyzing 104.

According to the method 100, the calculation of the refraction parameters of the eye is fast, robust and accurate. For example, the time to find the refraction parameters of the eye of an individual with the method 100 takes approximately fifty milliseconds, whereas the methods based on optimization of a model take approximately a few tens of seconds or is of the order of ten seconds to one minute. Thus, the computation time needed by the method of the invention is minimized.

In a particular embodiment of the method 100, it is possible to acquire an eccentric photorefraction image using simultaneously two light sources along two distinct meridians. Thus, in that embodiment, each eccentric photorefraction image corresponds to an image acquisition using two light sources of the plurality of light sources. In this particular embodiment, the two light sources used simultaneously are positioned on two distinctive positions, preferably along two distinctive meridians.

Figure 2 shows a second example of a method 200 according to the invention. The method 200 comprises all the steps of the method 100 shown in figure 1. Thus, only the differences between figure 1 and figure 2 will be described.

Before the step of analyzing, the method 200 comprises a pre-processing step 202. The pre-processing step comprises an image recognition step 204 configured to detect or discriminate elements from the eccentric photorefraction images allowing to detect and select elements, preferably at least one element of interest from the eccentric photorefraction images acquired in the step of acquiring 102. In this example, the elements, which have been selected in the image recognition step 204 are relative to areas of the eccentric photorefraction images in order to select, for example, the eye 1 of the individual. In addition, the image recognition step enables to detect the pupil of the eye and the eyebrow.

The method 200 illustrated in figure 2 further comprises a cropping step configured to select at least one of elements detected or discriminated in the image recognition step 204. In this example, the cropping step is included in the preprocessing step 202.

In this example, the images at the exit of the preprocessing step 202 are used as input in the step of analyzing 104 allowing to provide a step of analyzing 104 less time consuming because the elements of interest extracted from the eccentric photorefraction images have been selected. Thus, the neural network does not need to select the elements of interest by its own. The method 200 is thus optimized as to computation time.

For example, figure 3 shows an example of a preprocessing step 202 used in the method 100 and 200.

In this example, an eccentric photorefraction image is acquired. The eccentric photorefraction image comprises the face or a part of the face of the individual. Then, the recognition step 204 is performed. A part of one of the eyes is recognized in the image recognition step 204. For example, the eye 1a is detected as well as its eyebrow 2, a dark crescent in the pupil 3, and a bright part 4 extending up to the edge of the pupil 3. When the zone of interest is detected, for example the part 5 comprising at least the bright part 4 and the pupil 3 of the eye 1a of the individual, the cropping step 206 is carried out by selecting a small vignette 5 of the eccentric photorefraction image. The vignette 5 preferably comprises the pupil 3 and the bright crescent 4 of the eye. In another embodiment, the cropping step only selects the bright crescent 4 or the dark crescent. In a particular embodiment, the step of pre-processing 202 is adapted to measure a pupil diameter of the pupil 3 selected in this step. This vignette 5 is then provided at the input of the neural network used in the step of analyzing 104.

Of course, the pre-processing step 202 can be performed on the other eye 1b of the individual, alternatively or simultaneously. The pre-processing step 202 is preferably performed using an image processing algorithm. In another embodiment, the pre-processing step 202 uses a neural network, that is preferably different from the neural network used in the step of analyzing 104. In another embodiment, the neural network used in the step of analyzing is the same than the neural network used in the pre-processing step 202.

In the example of the method 200, the neural network is not built. The method 200 further comprises a training step 208 before the step of analyzing 104 in order to train the at least one neural network based on a set of training eccentric photorefraction images stored in a database. The training step 208 allows to build the neural network. In the training step 208, each training eccentric photorefraction image of the set of training eccentric photorefraction images is associated with a predetermined value for each of refraction parameters (in this example, the sphere value and the astigmatism parameters are considered). Thus, it means that the at least one refraction parameter associated to each image of the set of training eccentric photorefraction images is known. Of course, in another embodiment, only the sphere value is considered. Training is done for example using a method of retro-propagation of the gradient, by modifying the weights of the connections of the neural network, to minimize the mathematical distance at each iteration.

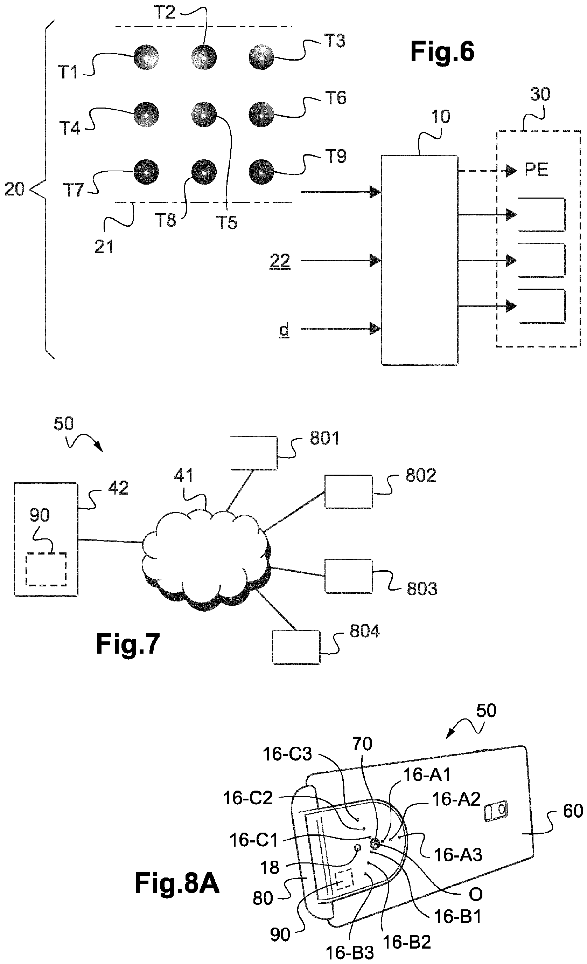

Then, the method 200 comprises an evaluation step 210 to test the at least one neural network using a set of test eccentric photorefraction images stored in the same or another database. A criterion is used to test the neural network. The criteria is based for example on the mathematical distance between the expected values and the values calculated by the current neural network. The evaluation step 210 consists in minimizing this distance. In the step of evaluating 210, each test eccentric photorefraction image of the set of test eccentric photorefraction images is associated with a predetermined value for each of refraction parameters. It allows to test the neural network. In addition, the set of training eccentric photorefraction images and the set of test eccentric photorefraction images are stored in a same memory of a remote server (see figure 7). The set of training eccentric photorefraction images and the set of test eccentric photorefraction images are different sets of images. Specifically, the set of test eccentric photorefraction images are images, which are not known by the neural network, allowing to test in an efficient manner the neural network after the training step 208. In another embodiment, the criteria used in the evaluation step 210 is based, for example, on mean error, or standard deviation. A predetermined threshold is fixed. For example, if the criterion is below a fixed value of threshold, the training step 208 is repeated with, for example, other set of training eccentric photorefraction images, and/or the architecture (design) of the neural network is changed, for example by adding new layers.

In another embodiment, the training step 208 stops after a fixed number of iterations. The evaluation of the performance of the neural network (in the evaluation step 210) is based on an estimation of the mean error and the standard deviation of at least one refraction parameter obtained at the output of the neural network, and then by comparing the expected values of the at least one refraction parameter with the calculated values obtained with the neural network in the step of training. As an example, a neural network is validated if the mean error is inferior to 0,1 diopter (D) for spherical equivalent with a standard deviation inferior to 0,7 D.

In addition, the set of training eccentric photorefraction images and the set of test eccentric photorefraction images are in a first embodiment acquired images. Thus, the method 200 optionally comprises a preliminary step of acquiring 212 before the step of training 208 and the step of evaluating 210.

The preliminary step of acquiring the set of training eccentric photorefraction images and the set of test eccentric photorefraction images is performed, in this example, with the same capturing device, which is used in the step of acquiring 102 the eccentric photorefraction images of the eye of the individual. Thus, it allows to get real images of the eye by the image capturing device. A photorefraction module is preferably associated to the image capturing device to determine the objective refraction parameters associated to each acquired image by the image capturing device used in the training and evaluation steps 208, 210. Preferably, the set of training eccentric photorefraction images and the set of test eccentric photorefraction images are images obtained with different individuals allowing to improve the accuracy of the neural network. In addition, the preliminary step of acquiring 212 the set of training eccentric photorefraction images and the set of test eccentric photorefraction images is performed for the two eyes of the individuals.

Thus, in this example, the set of training eccentric photorefraction images and the set of test eccentric photorefraction images are acquired images. For example, in this first embodiment, a plurality of eccentric photorefraction images is acquired by the image capturing device and then randomly divided into two groups to form the set of training eccentric photorefraction images and the set of test eccentric photorefraction images. Then, these sets of images are used separately in the training step 208 and respectively in the evaluation step 210. In addition, this preliminary step of acquiring 212 advantageously comprises a processing step 214. This processing step 214 is preferably equivalent to the pre-processing step 202 carried out before the step of analyzing 104.

In a second embodiment of the method 200, the set of training eccentric photorefraction images and the set of test eccentric photorefraction images are images obtained from a simulation. It means that the set of training eccentric photorefraction images and the set of test eccentric photorefraction images are not acquired images but simulated images. In that case, the method 200 comprises a simulation step 216 to simulate eccentric photorefraction images based on parameters. Using eccentric photorefraction images that are simulated allows to check the neural network because the results returned by the neural network can be compared with the parameters used as input to simulated the eccentric photorefraction images of the training and evaluating steps 208, 210.

The set of training eccentric photorefraction images and the set of test eccentric photorefraction images, which are simulated, are calculated by a calculation module or a processor, or a computer. In a particular embodiment, the calculation module used to simulate these images is a simulator. The simulator is configurated to simulate these sets of images allowing to generate a huge volume of simulated images of the eye with "theoretical" refraction parameters. By theoretical, it is meant that the refraction parameters of each simulated image are equal to the refraction parameters input into the simulator to simulate the sets of images.

For example, a method used to simulate the set of training eccentric photorefraction images and/or the set of test eccentric photorefraction images comprises an initialization step, for initializing parameters such as sphere, cylinder and axis values for the eye that are targeted; a step setting values for parameters (parameters related to the eye sought including at least sphere, cylinder and axis, and optionally high order aberrations or HOA, hardware related parameters and measurement parameters, such as measuring distance); a step of generating a set of simulated images based on the values for parameters set at the step of setting values. To improve this simulation, the method of simulation can further comprise a step of comparing the set of simulated images with the parameters of the eye set at the step of setting values or with real images used as reference images, for example a set of acquired eccentric photorefraction images of the eye; and a step of optimizing or minimizing an estimator of a difference between the set of simulated images and the set of target images (reference images) or the set of parameters used in the setting values step. The steps can be iterated until a minimum is found by using current optimization algorithm. The minimum corresponds to the best simulated image with the set of input parameters set in the step of setting values.

The datasets for low order aberrations and high order aberrations generated by the simulation model are based on documented statistical repartition of the refractive errors in the population. For instance, the following publications provide such statistics : Linda Lündström et al., J. Opt. Soc. Am. A 26, 2192-2198 (2009), Salmon & Van de Pol, Corina., Journal of cataract and refractive surgery (2007).

In a particular aspect, the set of training eccentric photorefraction images and the set of test eccentric photorefraction images include more one hundred thousand images.

In the example disclosed in figure 2, the method 200 comprises the preliminary step of acquiring 212 and the step of simulating 216. It means that the set of training eccentric photorefraction images and the set of test eccentric photorefraction images are a mix of real images obtained after the preliminary step of acquiring 212 with simulated images obtained after the simulating step 216. Preferably, the mix of images is of fifty percent of real images and fifty percent of simulated images. Of course, this embodiment is not limited to this percentage and the percentage of the mix can vary, for example, it can be of thirty percent or forty percent of real images and seventy or sixty percent of simulated images. Working with simulated and real eccentric photorefraction images improves the performances of the neural network because the neural network learns on two types of images. The neural network is thus more robust to the differences between simulated images and real images. By real, it is meant acquired images.

In the example of figure 2, the set of training eccentric photorefraction images and the set of test eccentric photorefraction images each comprise two thousand images. The neural network is improved by increasing the eccentric photorefraction images used in the training step 208.

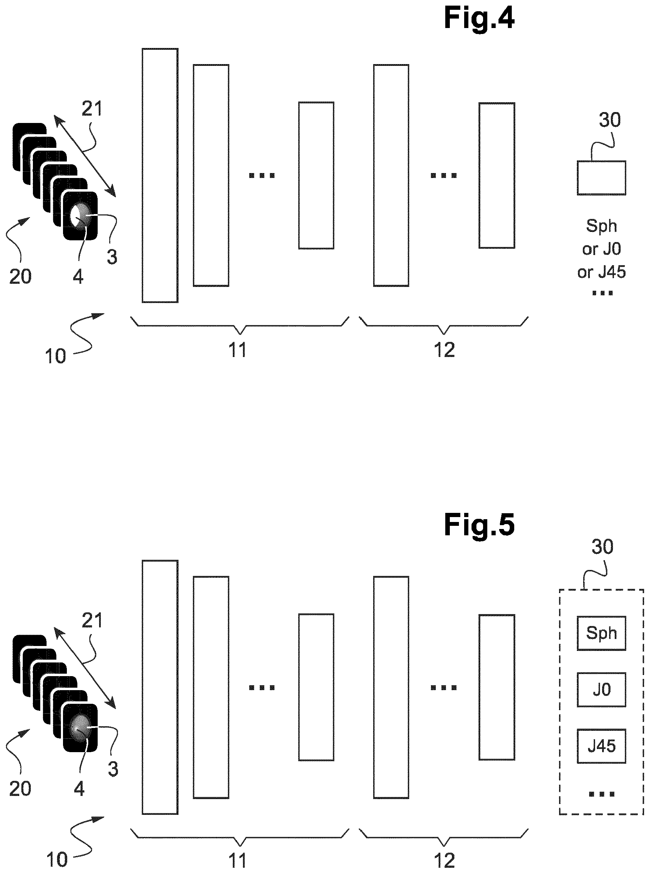

Figures 4, 5 and 6 show a representation of a neural network 10 used in the method 100 or 200.

The neural network 10 is a convolutional neural network 10.

For example, a typical design of the convolution neural network can be the following. A pattern based on a plurality of Conv-Relu layers with a Pool layer is repeated to obtain a desired vector (i.e. small vector), then the convolutional neural network is ending by two connected layers, one generally called fully connected layer (FC layer) and the last one (output layer). The pattern can be written as [(Conv → Relu)ₚ → Pool]ₙ with n, p corresponding to a number of repetition of these layers.

In this example, the neural network comprises at least three convolutional layers and at least two output layers 12.

Thus, in the convolutional neural network 10, the at least three convolutional layers correspond to a repetition of one pattern and the at least two output layers correspond to the FC layer and the output layer.

The neural network used in the method 100 or 200 comprises input parameters 20. In the examples of figure 4 and 5, the neural network 10 comprises, at its input, a set of eccentric photorefraction images 21 that have been acquired in the step of acquiring 102. The set of eccentric photorefraction images 21 shows the pupil 3 of the eye the individual and the crescent 4 comprised in the pupil 3. The set of photorefraction images 21 is, for example, images obtained after the preprocessing step 202 as shown in figure 3.

Then, the neural network 10 shown in figures 4 and 5 is configurated to provide at its output refraction parameters 30 of the eye of the individual.

In the example of figure 4, the neural network 10 provides a sphere value noted Sph or one of the astigmatism features, J0 or J45. The neural network 10 in this example is associated to a single refraction parameter, such as a sphere value noted Sph. In the example of figure 4, the method 100 or 200 assigns a neural network 10 for each desired refraction parameters. Thus, it means that if other refraction parameters are desired, for example, if the method 100 or 200 must find astigmatism features, such as a cylinder and an axis, two other neural networks 10 need to be used by the method 100 or 200.

In contrast, the neural network 10 of the example of figure 5 provides the sphere value Sph and astigmatism features simultaneously. In this example, the astigmatism features comprise cylinder and axis features, represented by J0 and J45. Thus the neural network 10 shown in figure 4 is a single neural network 10 for all the refractions parameters 30. The output of the neural network 10 is here a vector comprising, for example, the sphere value and the astigmatism features.

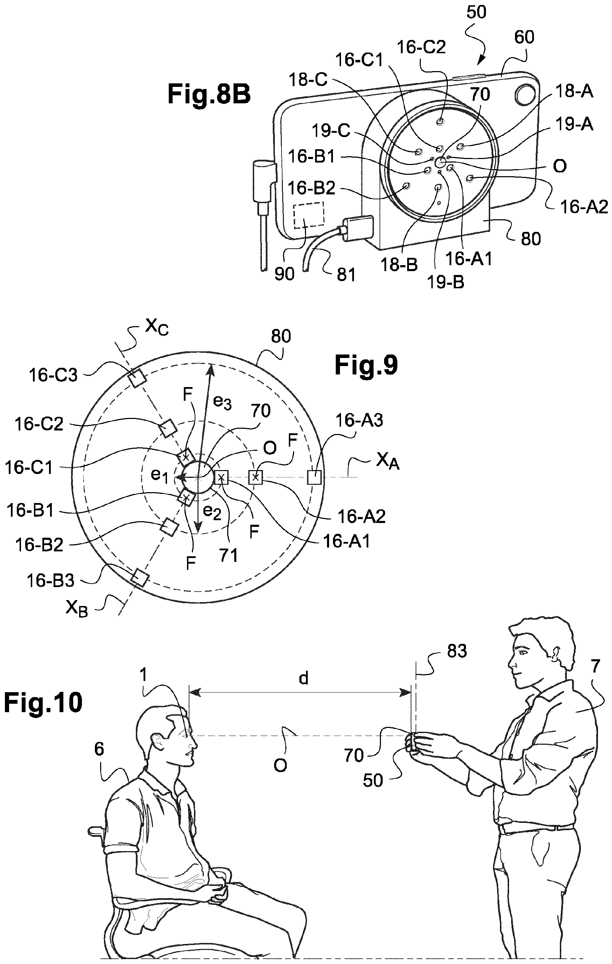

The example of figure 6 shows another embodiment of neural networks 10. In this example, the neural network 10 comprises other input parameters. The input parameters comprise the set of eccentric photorefraction images 21, a set of positions 22 comprising a position of each light source belonging to the plurality of light sources used in the step of acquiring 102 and a distance, noted d and corresponding to the distance between the image capturing device and eye of the individual during the step of acquiring 102. The set of eccentric photorefraction images 21 comprises, in this example, nine acquired eccentric photorefraction images and noted T1 to T9. Preferably, the position 22 of each light source comprises an angle and an eccentricity (see figures 8A, 8B, 10). For example, the distance d is measured using a time of flight device.

The neural network 10 of figure 6 provides the same refraction parameters as the neural network of figure 5. In addition, the neural network of figure 6 is configured to further provide a pupil diameter, noted PE (represented as 2r on figure 3), of the eye of the individual. In another embodiment, the size of the dark crescent 4 is also determined (noted s on figure 3). Thus, it means that the neural network 10 of figure 6 is configured to determine the pupil diameter PE of the eye of the individual from the set of eccentric photorefraction images 21 provided at its input.

### Device

Figures 8A and 8B represent a device 50 for estimating refraction of an eye 1 of a subject. The system 50 comprises a photorefraction module 80, or addon, linked to a mobile device 60 such as a smartphone, a tablet personal computer or a laptop computer. Advantageously, the photorefraction module 80 is mechanically fastened to the mobile device 60. In another embodiment, the device 50 comprises a photo retinoscopy module 80 in the place of the photorefraction module 80.

The photorefraction module 80 is connected to the mobile device 60 using a direct plug and socket connection as illustrated on figure 8A or using a wire connection 81 as illustrated on figure 8B. The photorefraction module 80 is configured to communicate with the mobile device 60 either using a wired connection or a wireless connection.

The photorefraction module 80 illustrated on figures 8A, 8B and 10 comprises an image capturing device 70, also called camera 70, and a plurality of P light sources, wherein P is an integer higher than or equal to two. In other words, the minimum configuration comprises at least two light sources, 16-A and 16-B, arranged along two distinct meridians, X_{A} and X_{B}. The camera 70 is preferably arranged to provide high-resolution eccentric photorefraction images, allowing to improve the accuracy of the step of analyzing 104.

For example, the photo refraction module/add-on is linked to a smartphone.

For example, in figure 9, the photorefraction module 80 comprises nine light sources 16-A1, 16-A2, 16-A3, 16-B1, 16-B2, 16-B3, 16-C1, 16-C2 and 16-C3. The three light sources 16-A1, 16-A2, 16-A3 are arranged along a same direction, or meridian X_{A}, transverse to the optical axis, noted O, of the camera 70. Similarly, the three light sources 16-B1, 16-B2, 16-B3, respectively 16-C1, 16-C2, 16-C3, are arranged along another direction, or meridian X_{B}, respectively meridian X_{C}, The meridians X_{A} X_{B} and X_{C} are transverse to the optical axis O of the camera 70. The three meridians X_{A}, X_{B} and X_{C} correspond to three distinct directions in a same plane 83 perpendicular to the optical axis O of the camera 70 (see figure 10). For example, the three meridians X_{A}, X_{B} and X_{C} are separated by an angle of 120 degrees from each other. In another embodiment (not shown), the light sources are arranged along to two meridians transverse to the optical axis O of the camera 70. The two meridians can be separated by an angle inferior or equal to 180 degrees.

As an option, the photorefraction module 80 further comprises another light source 18 (see fig. 8A). Alternatively, the photorefraction module 80 further comprises another set of three light sources 18-A, 18-B, 18-C arranged at another eccentricity and placed on three other meridians and still another set of three light sources 19-A, 19-B, 19-C arranged at another eccentricity (close to the edge of the camera 70) and placed on the same or three other meridians, as illustrated on Figure 8B. Advantageously, as illustrated on Fig. 8B the photorefraction module 80 comprises twelve light sources, for example 12 LEDs. According to another alternative, the photorefraction module 80 further comprises another set of three light sources 18-A, 18-B, 18-C arranged on another and same meridian and placed at three different eccentricities from the camera.

In the example of figure 8, an angle of 180 degrees separates the light source numbered 16-B2 from the light source numbered 18-A. In addition, the light source numbered 16-C1 is separated from the light source numbered 18-A by an angle of at least 3 degrees.

In figure 8A, the pluralities of light source 16, 18 are at a distance comprised between 0,3 millimeters and 20 millimeters from an edge of the camera 70. For example, a distance of 0,3 millimeter separates a light source center, noted F, of the light sources 16-A1, 16-C1, 16-B1 from the edge 71 of an aperture of the camera 70. This photorefraction module 80 enables to measure very small values of photorefraction thanks to the small eccentricities of the three light sources 16-A1, 16-B1, 16-C1 with respect to the optical axis of the camera. Thus, this photorefraction module 80 enables to measure very small values of refraction (sphere, cylinder and/or axis). The larger the number of light sources and the number of meridians the higher the accuracy of the results.

The combination of the photorefraction module 80 with a neural network enables to obtain both high sensitivity and high accuracy refraction measurements while using a much shorter computation time as compared to conventional optimization methods based on a model.

In an example, the set i of light sources 16-Ai, 16-Bi, 16-Ci emits light at a first wavelength and the set of at least another light source 18, respectively 18A, 18B, 18C, emits light at a second wavelength, distinct from the first wavelength. Generally, the first wavelength is in the near infrared or infrared range, for example around 850 nm, so that the pupil of the user remains unchanged when the light source is lit up. In this embodiment, 10 nanometers (nm) separate the first wavelength from the second wavelength, when for example the second wavelength is of 860 nanometers. In a preferred embodiment the set i of light sources 16-Ai, 16-Bi, 16-Ci and the set of of light sources 18-A, 18-B, 18-C are similar. Thus, it means that all the light sources 16, 18 emit at the same wavelength. In this example, all the light sources 16, 18 comprise a wavelength of 850 nanometers.

The position of each light source 16, 18, 19 relatively to the camera 70 is predetermined and fixed. Each set *i* of light sources 16-Ai, 16-Bi, 16-Ci is placed at a same distance, or eccentricity *eᵢ*, from the optical axis 0 of the camera 70. The range of eccentricity is generally comprised between 1 and 300 mm or 500 millimeters. Advantageously, the range of eccentricity is generally comprised between 1 mm to 20 mm.

Advantageously, the light sources consist of light emitting diodes or leds. For example, the photorefraction module 80 comprises nine leds arranged at three different eccentricities along three different meridians. The camera 70 is adapted and configured for capturing eccentric photorefraction images of the eye 1 of the individual for each light source 16-Ai, 16-Bi, 16-Ci for i = 1, 2, 3 that is lit successively. This configuration enables to acquire a set of N=9 eccentric photorefraction images per measurement. For example, the light sources 16, 18 provide the set of eccentric photorefraction images 21 illustrated on figure 6 and numbered T1 to T9. Each eccentric photorefraction image is associated to one and only one light source of the plurality of light sources.

In another example, the photorefraction module 80 comprises twelve light sources arranged along three meridians and at four different eccentricities.

The position of the light sources 16, 18, 19 allows to scan spatially and angularly the eye 1 of the individual over multi-directions. Thus, the evaluation of the refraction of the eye is a multidirectional evaluation, allowing to provide accurate evaluation of the refraction parameters of the eye of the individual. In addition, the method is easily to implement because there is no need to rotate the device 50 to obtain the evaluation over multi-directions.

In another embodiment, it is possible to acquire an eccentric photorefraction image 21 using simultaneously two light sources along two distinct meridians. In that embodiment, an eccentric photorefraction image is acquired with 2 LEDs lit on simultaneously. The eccentric photorefraction image of the pupil 3 of the eye comprises in that embodiment two distinctive crescents 4, each being associated to a light source. The two LEDs are preferably arranged along two different meridians to avoid superposition of the two crescents. For example, the light source used in this embodiment could be the first light source numbered 16-A1 positioned on the meridian X_{A} and the second light source could be the light source numbered 16-C2 and positioned on the meridian X_{C} on figure 9. For example, a first bright crescent is generated by the first light source 16-A1 (LED) placed along meridian X_{A} and a second bright crescent 52 is generated by the second light source 16-C2 (LED) placed along meridian X_{C.} Each bright crescent is typically delimited by an abrupt transition curve between a high grey level and a low grey level in the eccentric photorefraction image. Thus, it is possible to determine the size of two dark crescents from a single eccentric photorefraction image.

More generally, it is possible to take a set of eccentric photorefraction images 21, with two LEDs lit on simultaneously to generate 2 crescents in the pupil 3. Preferably the two light sources are arranged on 2 different meridians. For example, a first image is captured using two LEDs numbered 16-A1 and 16-C2 ; a second image is captured using two other LEDs numbered 16-B1, 16-A2; and a third image is captured using two other LEDs numbered 16-A2, 16-C3 (see figure 9). All the combinations of the light sources belonging to the device 50 could be implemented as long as the two light sources used to acquire simultaneously a same eccentric photorefraction image are arranged on two different meridians. Thus, two light sources of same eccentricity can be used as long as they are positioned along two different meridians.

Figure 10 shows a configuration for estimating refraction of an eye 1 of an individual 6 using a device 50 according to the invention. An optometrist 7 or a technician holds the device 50 and arranges the position and direction of the optical axis of the camera 70 so as to be at the height of the face of the individual and so that the plane 83 of the light sources 16, 18 is vertical. The photorefraction module 80 is at a distance d from the eye 1 of the individual 6. Generally, the distance d is about 1 meter, and can be comprised between 30 cm and 2 m. In an example, we consider two distance measurement values: 1m and 0,5m. Thus, in case the measurement at 1 m returns no accurate result, the measurement is performed at the distance of 50 cm. Advantageously, the device 50 comprises means for measuring the distance d, such as an autofocus device. Alternatively, the subject 6 holds the device 50 and looks at the camera directly or by reflection on a mirror surface and takes the pictures himself for self-refraction measurement. The device 50 is very tolerant in positioning and non-invasive.

The device 50 also comprises a calculation module 90. The calculation module preferably comprises a memory and a processor comprising a memory and a processor, arranged to execute program instructions stored in the memory. The neural network is preferably stored in the memory of the calculation module 90. In a first embodiment, the calculation module 90 is placed inside the photorefraction module 80 (see figure 8A) or into the mobile device 60 (See Figure 8B). When the calculation module 90 is placed inside of the mobile device 60, the neural network 10 is stored in the memory of the mobile device 60. Thus, the method according to the invention is easy to use and implement because it works with common devices. Alternatively, the calculation module 90 is placed inside a remote computer in communication with the photorefraction module 80 (see figure 7). The calculation module 90 is configurated to carry out the step of analyzing 104 of the method 100 or 200 to determine the photorefraction parameters of the eye 1 of the individual 6, preferably of each eye of the individual 6.

In this example, the photorefraction module 80 is used to perform an objective refraction by analyzing the size, shape and orientation of the bright part of the pupil of the eye 1 of the individual when the eye 1 is lit up by a light source of the plurality of light sources and observed by the camera 70. Preferably, each light source is an eccentric flash. This analysis is performed in the step of analyzing 104.

In these embodiments, it is noted that the photorefraction module 80 and the image capturing device 70 are embedded into a same device, the photorefraction module 80.

Figure 7 shows another schematic representation of a device 50 according to the invention.

The device 50 comprises four photorefraction modules 80, respectively numbered 801, 802, 803 and 804 which are connected to internet 41. Each photorefraction module 80 comprises a plurality of light sources 16, 18 and an image capturing device 70. The device 50 comprises a remote server 42 connected to the internet 41. Thus, the remote server 42 is connected via internet 41 to each photorefraction module. Each photorefraction module is linked to a mobile device 60 as shown on figure 8A.

In this example, the remote server 42 is used to carry out the step of analyzing 104, the step of training 208 and the step of evaluating 210.

The remote server 42 shown in figure 7 comprises the calculation module 90 in which the neural network 10 is stored. Thus, the step of analyzing 104 is carried out remotely.

The step of acquiring 102 is carried out by the camera 70 of the photorefraction module 80. The remote server 42 is arranged to receive the eccentric photorefraction images T1-T9 from the photorefraction module 80 and then carry out the step of step of analyzing 104, or the step of training 208 or the step of evaluating 210.

A remote server 42 is suitable for the training step 208 and the evaluating step 210 when a large amount of training eccentric photorefraction images and test eccentric photorefraction images are used.

In another embodiment, the calculation module 90 belongs to a remote computer in communication with the photorefraction module 80. In this embodiment, the remote server 42 is used as a database to perform the step of training and evaluating 208, 210, whereas the step of analyzing 104 is performed by the remote computer connected to the refraction module 80 via internet 41.

## Claims

1. Method for estimating refraction of an eye of an individual using an image capturing device, said image capturing device having an optical axis and being placed at a distance d from the eye of the individual, the method comprising the following steps:
- acquiring eccentric photorefraction images of the eye of the individual using the image capturing device when the eye is successively illuminated by a plurality of light sources, each eccentric photorefraction image corresponding to an image acquisition using at least one light source of the plurality of light sources;
- analyzing the eccentric photorefraction images by a calculation module in order to determine at least one refraction parameter comprising a sphere value;
**characterized in that** the step of analyzing is carried out by machine learning using at least one neural network configured to determine the at least one refraction parameter from the eccentric photorefraction images provided to the calculation module, the plurality of light sources in the step of acquiring being positioned at at least two different eccentric distances from the optical axis of the image capturing device and/or arranged along at least two different directions transverse to the optical axis of the image capturing device.

2. Method according to claim 1 wherein the neural network is configured to determine the at least one refraction parameter based on the acquired eccentric photorefraction images and based on a set of inputs representing at least the distance d, and a position of each light source of the plurality of light sources relatively to the image capturing device.

3. Method according to claim 1 or 2 wherein the method comprises a training step before the step of analyzing in order to train the at least one neural network based on a set of training eccentric photorefraction images stored in a database and an evaluation step to test the at least one neural network using a set of test eccentric photorefraction images stored in the same or another database, wherein each training eccentric photorefraction image of the set of training eccentric photorefraction images, and respectively, each test eccentric photorefraction image of the set of test eccentric photorefraction images, is associated with a value for each of the at least one refraction parameter.

4. Method according to claim 3 wherein the set of training eccentric photorefraction images and the set of test eccentric photorefraction images comprise images acquired using the image capturing device and/or simulated images obtained using a simulation model.

5. Method according to any one of claim 1 to 4 wherein the method comprises a pre-processing step comprising an image recognition step configured to detect or discriminate elements from the eccentric photorefraction images, said elements being relative to areas of the eccentric photorefraction images.

6. Method according to claim 5 wherein the method further comprises a cropping step configured to select at least one of elements detected or discriminated in the image recognition step.

7. Method according to any one of claim 1 to 6 wherein the at least one refraction parameter further comprises at least one other parameter of the eye among: astigmatism features, higher order aberrations, and/or wherein said step of analyzing further determining at least one individual parameter among: pupil diameter of the eye of the individual, half interpupillary distance, direction of gaze, amount of red reflex and Stiles-Crawford parameter.

8. Method according to claim 7 wherein the at least one neural network used in the step of analyzing comprises a different neural network for each refraction parameter or a single neural network for all the refraction parameters of the at least one refraction parameter.

9. Method according to claim 1 to 8 wherein the at least one neural network comprises a convolutional neural network comprising at least three convolutional layers and at least two output layers.

10. Device for estimating refraction of an eye of an individual, said device for estimating refraction comprising:
- a plurality of light sources arranged to successively illuminate the eye of the individual;
- an image capturing device having an optical axis, the image capturing device being placed at a distance d from the eye of the individual, the image capturing device being arranged for capturing eccentric photorefraction images of the eye of said individual, each eccentric photorefraction image being associated to at least one light source of the plurality of light sources;
- a calculation module arranged to analyze the eccentric photorefraction images in order to determine at least one refraction parameter comprising a sphere value;
**characterized in that** the calculation module is arranged to analyze the eccentric photorefraction images by machine learning using at least one neural network configured to determine the at least one photorefraction parameter, said plurality of light sources being positioned at at least two different eccentric distances from the optical axis of the image capturing device and/or arranged along at least two different directions transverse to the optical axis of the image capturing device.

11. Device according to claim 10 wherein at least one light source of the plurality of light sources is at a distance comprised between 0,3 millimeter and 20 millimeters from an edge of the image capturing device.

12. Device according to any one of claim 10 to 11 wherein each light source is placed at a distance from the other light sources comprised between 1 millimeter and 300 or 500 millimeters or spaced from the other light sources of an angle comprised between 3 degrees and 180 degrees, preferably between 3 degrees and 120 degrees, said angle being defined according to two different directions of two light sources of the plurality of light sources with respect to the optical axis of the image capturing device.

13. Device according to any light sources is arranged to emit at a wavelength in the near infrared or infrared one of claim 10 to 12 wherein the calculation module is embedded into a mobile device attached to the image capturing device or stored in a remote server.

14. A computer-program product comprising one or more stored sequences of instructions that are accessible to a processor, and which, when executed by the processor, causes the processor to carry out the method according to any one of claim 1 to 9.

15. A computer readable medium carrying one or more sequences of instructions of the computer program product of claim 14.
